Europäisches Patentamt

European Patent Office

Office européen des brevets

(19)

(11) Numéro de publication: **0 184 514**
**A2**

## (12) DEMANDE DE BREVET EUROPEEN

(21) Numéro de dépôt: 85402401.5

(22) Date de dépôt: 04.12.85

(51) Int. Cl.⁴ **A61K 31/315**

(30) Priorité: 07.12.84 FR 8418755

(43) Date de publication de la demande:
**11.06.86 Bulletin 86/24**

(84) Etats contractants désignés:
**BE CH DE GB IT LI**

(71) Demandeur: SOCIETE CIVILE DE RECHERCHES ET
D'ETUDES THERAPEUTIQUES
33, Avenue Arouet
F-92160 Antony(FR)

(72) Inventeur: **Suck, Catherine**
**10, Rue Emile Morel**
**F-92330 Sceaux(FR)**
Inventeur: **Chazot, Guy**
**31, Quai Jean-Jacques Rousseau**
**F-69350 La Mulatiere(DE)**

(74) Mandataire: **Combe, André et al**
**CABINET BEAU DE LOMENIE 55 rue d'Amsterdam**
**F-75008 Paris(FR)**

(54) Médicaments à base de gluconate de zinc utiles pour le traitement de la sclérose en plaques.

(57) La présente invention concerne l'utilisation du gluconate de zinc pour la fabrication d'un médicament administré par voie orale et contenant de 50 à 150 mg de gluconate de zinc, ledit médicament étant utilisé pour le traitement de la sclérose en plaques.

EP 0 184 514 A2

Médicaments à base de gluconate de zinc utiles pour le traitement de la sclérose en plaques.

La présente invention concerne un nouveau médicament fortement dosé en gluconate de zinc utile pour le traitement de la sclérose en plaques.

On a déjà préconisé l'utilisation de sels de zinc, notamment les acétate, chlorure, sulfate ou gluconate pour le traitement de certaines maladies. C'est ainsi qu'il existe sur le marché des ampoules contenant chacune 0,5 mg environ de sel de zinc, éventuellement associé à des sels de cuivre ou éventuellement à des sels de nickel et de cobalt.

On a par ailleurs récemment publié (revue de médecine n° 32, octobre 1982) un article remarquablement documenté faisant le point des connaissances sur le zinc, sa régulation physiologique chez l'homme et les troubles liés à son déficit ou à son excès. Dans cette publication, on suggère la possibilité d'administrer journellement à des patients des sels de zinc à dose de 10 à 45 mg de zinc par jour pour le traitement des états de carence nutritionnelle en zinc et en particulier pour le traitement de l'acrodermatite. Mais, dans la pratique, il semble que l'on ne trouve pas aujourd'hui sur le marché de médicaments permettant l'administration de zinc en hautes quantités.

Il a été trouvé maintenant, et c'est là l'objet de la présente invention, que les médicaments nouveaux, administrés par voie orale, et contenant par dose unitaire de 50 à 150 mg de gluconate de zinc, sont utiles pour le traitement de la sclérose en plaques.

L'invention est ainsi fondée sur les deux constatations suivantes :

- la remarquable tolérance gastrique - plus généralement sur la plan digestif - du gluconate de zinc, même lorsqu'il est administré à des doses élevées,

- le fait qu'une maladie telle que la sclérose en plaques puisse être sensible à un traitement à base de zinc.

Bien évidemment, l'inocuité de gluconate de zinc aux doses utilisées a été vérifiée chez le rat et chez l'homme.

Dans les médicaments selon l'invention, le gluconate de zinc est avantageusement en association avec un véhicule pharmaceutiquement acceptable. Ces médicaments peuvent être sous la forme de solutions, de comprimés ou de gélules, ces dernières étant la forme de présentation préférée. On indiquera à titre d'exemple qu'une posologie moyenne de 200 mg de gluconate de zinc, per os, en deux prises, convient pour le traitement de la sclérose en plaques.

Le traitement de la sclérose en plaques par le gluconate de zinc à des doses élevées doit être considéré essentiellement comme un traitement de prévention et/ou d'entretien, c'est-à-dire, un traitement suivi régulièrement pendant plusieurs mois.

Les exemples suivants illustrent l'utilisation du gluconate de zinc à haute dose.

Exemple 1 - Madame B

Madame B... est âgée de 24 ans, elle a présenté à l'âge de 20 ans, une névrite optique rétro-bulbaire à droite. L'histoire actuelle remonte au 10.9.82 où la malade présente un syndrome cérébelleux vestibulaire et également pyramidal au membre inférieur droit. On note sur le plan sensitif un déficit sensitif à tous les modes au membre supérieur gauche. La ponction lombaire montre 0,42 de protéines totales, 7 éléments par mm³ et des I.g.G. à 18%.

La malade reçoit une cure aigüe de corticoïdes puis est mise à un traitement préventif par le gluconate de zinc à raison d'une ampoule à 100 mg à 10 h et 18 h en dehors des repas. La malade est revue régulièrement tous les mois. L'évolution est très favorable puisque, examinée le 3.4.83, il n'existe plus ni syndrome cérébelleux, ni syndrome vestibulaire, ni syndrome pyramidal et les troubles sensitifs ont totalement disparu.

Tolérance : La tolérance est excellente sur le plan digestif. aucune complication n'est notée. On doit préciser que deux mois après l'institution de la thérapeutique une ponction lombaire est réalisée, montrant une normalisation du liquide céphalo-rachidien, protéinorachie 0,34, moins d'un élément par mm³, I.g.G. 10%.

Exemple 2 - Madame C

Madame C... est âgée de 38 ans, elle est suivie depuis 5 ans pour sclérose en plaques évoluant par poussées très fréquentes avec une poussée par mois environ. Il s'agit d'une forme ayant associé initialement un syndrome cérébelleux, un syndrome pyramidal, des troubles sensitifs et une névrite optique rétro-bulbaire. Lors de l'hospitalisation, au mois de septembre 82, la symptomatologie est dominée par des phénomènes paresthésiques et dysesthésiques au niveau de l'hémicorps gauche, survenant mensuellement. Cette survenue a conduit à des traitements prolongés au Synactène Retard qui a entraîné des effets secondaires importants. La malade est mise au gluconate de zinc, 100 mg à 10 h et à 18 h. Il n'y a pas d'autre thérapeutique associée. Avec un recul de six mois, on constate une disparition des phénomènes paresthésiques survenant chaque mois, l'examen neurologique montrant la persistance d'un signe de Babinski bilatéral sans déficit moteur.

Tolérance : La tolérance digestive est excellente. Aucun effet secondaire n'est signalé.

Exemple 3 - Madame G

Madame G... est âgée de 30 ans. Elle présente une sclérose en plaques évoluant par poussées ayant associé successivement paralysie faciale périphérique gauche, névrite optique rétro-bulbaire, syndrome pyramidal à droite, syndrome cérébelleux, syndrome vestibulaire et troubles sensitifs à type d'hypoesthésie du membre inférieur droit. Malgré des cures discontinues de corticoïdes et un traitement à l'Imurel, évolution à rechutes. Devant l'inefficacité de l'Imurel, une thérapeutique au gluconate de zinc est instituée à raison de 200 mg de gluconate de zinc à 10 h et à 18 h. Avec un recul de quatre mois, on constate qu'il n'y a pas eu de nouvelles poussées, alors que les poussées précédentes étaient rapprochées tous les mois.

Ces trois exemples montrent à l'évidence que le gluconate de zinc, administré seul ou en association avec d'autres médicaments connus, constitue un médicament très utile dans le traitement de certaines scléroses en plaques : notons que des essais comparatifs ont pu mettre en évidence qu'une dose insuffisante de gluconate de zinc ne permettait pas d'atteindre le résultat recherché.

**Revendications**

1. Utilisation du gluconate de zinc pour la fabrication d'un médicament administré par voie orale et contenant de 50 à 150 mg de gluconate de zinc, ledit médicament étant utilisé pour le traitement de la sclérose en plaques.